# EUROPEAN PATENT SPECIFICATION

(11) **EP 1 565 208 B1**
(45) Date of publication and mention of the grant of the patent: **01.07.2009**
(21) Application number: 03754349.3
(22) Date of filing: 24.10.2003
(51) Int. Cl.: A61K 38/44, G01N 33/574, A61P 35/00

(54) **THE USE OF CYTOCHROME P450 ENZYME CYP2WI AS A DRUG TRAGET FOR CANCER THERAPY**
EINSATZ DES CYTOCHROM P450-ENZYMS CYP2WI ALS ZIELMOLEKÜL FÜR WIRKSTOFFE BEI DER KREBSTHERAPIE
UTILISATION DE L'ENZYME CYP2WI DU CYTOCHROME P450 COMME CIBLE DE MEDICAMENT EN CANCEROTHERAPIE

(30) Priority: 24.10.2002 SE 0203137; 24.10.2002 US 420787 P
(43) Date of publication of application: 24.08.2005
(73) Proprietor: Ingelman-Sundberg, Magnus, 182 33 Danderyd (SE); Wigzell, Hans, 12935 Hägersten (SE)
(72) Inventor: INGELMAN-SUNDBERG, Magnus, S-182 33 DANDERYD (SE); KARLGREN, Maria, S-169 72 SOLNA (SE); GOMEZ, Alvin, S-104 05 Stockholm (SE); HANS, Wigzell, SE-12935 Hägersten (SE)
(74) Representative: Lindgren, Anders
(86) International application number: PCT/SE2003/001652
(87) International publication number: WO 2004/037282

(56) References cited:
- WO-A1-01/58455
- DATABASE EMBL [Online] 01 June 2002 XP002988433 Database accession no. Q8TAV3
- YOUSSEF JOUNAIDI ET AL.: 'Frequent, Moderate-Dose Cyclophosphamide Administration Improves the Efficacy of Cytochrome P-450/Cytochrome P-450 Reductasebased cancer Gene Theraphy1' CANCER RESEARCH vol. 61, 01 June 2001, pages 4437 - 4444, XP002988432
- DATABASE CAPLUS [Online] XP002988472 Retrieved from STN Database accession no. 1997:210366 & OYAMA, TSUNEHIRO ET AL.: 'Cytochrome P450 2E1 polymorphism as a risk factor for lung cancer: in relation to p53 gene mutation' ANTICANCER RESEARCH vol. 17, no. 1B, 1997, pages 583 - 587
- DATABASE BIOSIS [Online] XP002988434 Database accession no. PREV200200495945 & ITOGA SAKAE ET AL.: 'Tandem repeat polymorphism of the CYP2E1 gene: An association study with esophageal cancer and lung cancer' ALCOHOLISM CLINICAL AND EXPERIMENTAL RESEARCH vol. 26, no. 8, August 2002, pages 15S - 19S
- DATABASE CAPLUS [Online] XP002988473 Retrieved from STN Database accession no. 1996:580855 & VASQUEZ, HERNAN G. ET AL.: 'Effect of cytochrome P450 inducers on expression of oncogenes and a tumor suppressor gene in human colon cancer cells' INTERNATIONAL JOURNAL OF ONCOLOGY vol. 9, no. 3, 1996, pages 427 - 431
- MAGNUS INGELMAN-SUNDBERG: 'Genetic susceptibility to adverse effects of drugs and environmental toxicants The role of the CYP family of enzymes' MUTATION RESEARCH vol. 482, no. 1-2, 10 January 2001, pages 11 - 19, XP008050822
- DANIEL W NEBERT ET AL.: 'Clinical importance of the cytochromes P450' THE LANCET vol. 360, 12 October 2002, pages 1155 - 1162, XP004387387

## Description

### Field of the invention

The present invention relates to a novel drug target in cancer therapy. More closely it relates to use of the cytochrome P450 enzyme CYP2W1 and its promoter as a drug target for cancer therapy. It also relates to therapeutic agents comprising moieties showing binding affinity for CYP2W1 and moieties with cytotoxic/anti-cancer effect.

### Background of the invention

The cytochromes P450 are responsible for the metabolism of endogenous as well as exogenous compounds. In human at present about 80 different P450 genes have been identified of which 55 are functional whereas 25 are pseudogenes. The human P450 forms can be divided into three major groups: i) those in CYP families 5-51 of endogenous importance having usually high affinity for the substrates and being relatively well conserved during evolution, ii) those in CYP families 1-3, with usually less affinity for their substrates, being less conserved evolutionary and which exhibit important genetic polymorphisms and iii) those in family 4 with properties in between the two other groups (see Ingelman-Sundberg (2002) for a review). CYP enzymes in families 1-3 are active in metabolism of exogenous compounds and currently 19 genes encoding active enzymes have been described. The cytochromes P450 in these families are responsible for 70-80 % of all phase I dependent metabolism of clinically used drugs and participate in the metabolism of a huge number of other xenobiotic chemicals. This can lead to the metabolic activation of precarcinogens and drugs, which might exert toxic or carcinogenic effects. This capacity to activate prodrugs to cytotoxic products are seen e.g. in the metabolic activation of acetaminophen which is metabolised by CYP2E1 and CYP3A4 to a reactive imine intermediate that can cause liver hepatotoxicity. Other examples of cytochrome P450 dependent metabolic activation come from prodrugs in cancer therapy where P450 enzymes like CYP2B6 inserted by viral transfection into tumours facilitate tumour suppression upon treatment with cancer drugs agents like e.g cyclophosphamide or ifosfamide (Waxman et al., 1999, Jounaidi and Waxman, 2001).

### Summary of the invention

The invention is based on the specific expression of the cytochrome P450 enzyme CYP2W1 in transformed tissue. This tissue specific expression found by the inventors can be used to target drugs and prodrugs to the transformed cells.
The invention thus relates to a compound comprising one part conferring cytotoxic and/or anti-cancer effects to the compound and one part conferring binding affinity towards CYP2W1 to the compound. It also relates to a pharmaceutical composition comprising such a compound.
In yet a further aspect the invention relates to the CYP2W1-promoter and use thereof in gene therapy.

The invention can for example be used for, but is not restricted to, the treatment of lung tumours, colon tumours or ovarian tumours, or all or any tumour expressing CYP2W1 constitutively or after administration of an inducing agent.

### Brief description of the figures

**Figure 1**
   Gene structure (A), nucleotide and predicted amino acid sequence (B) of CYP2W1. (A) Exons are shown as boxes with size in base pairs. The size of the introns is shown in kilo base pairs. (B) The stop codon is indicated by an asterisk and the proline-rich region, PPGP, and the conserved cysteine are shown in italic bold. The 3'-flanking region was obtained from the Celera Transcript hCT1786066.
**Figure 2**
   Unrooted phylogenetic tree of CYP2W1 and other human P450s belonging to family 1, 2 and 3. The scale bar for distance measurements represents 0.1 amino acid substitutions per site.
**Figure 3**
   Real-time PCR based quantification of CYP2W1 mRNA expression in Human Tumour Multiple Tissue cDNA (MTC) Panel using gene specific primers.
**Figure 4**
   Distribution of CYP2W1 mRNA in different human tissues and cell lines. (A) Multiple Tissue Expression (MTE) Array containing 42-500 ng of normalized human poly(A)⁺ RNA per dot. (B) Multiple Tissue Northern (MTN) blot containing approximately 1 µg poly(A)⁺ RNA per lane and Northern blot containing 20 µg total RNA from human liver, HepG2, HeLa, B16A2 or Heck293 cells per lane. All blots were hybridized with a ³²P-labeled probe corresponding to nucleotides 329-986 and exposed for 48 hours. The size markers used for the northern blot were the rRNA 28S (4.6-5.3 kb) and 18S (1.8-2.0 kb) band.
**Figure 5**
   Western blot containing four lanes with HepG2 total cell extract corresponding to 40 µg protein per lane. The first two lanes were incubated with CYP2W1 antiserum, while the last two were incubated with antiserum blocked with peptide. The blot was exposed for 3 minutes.
**Figure 6**
   CO-spectral analysis of the microsomal fraction of the pCMV4-2W1 II6 construct.
**Figure 7**
   Western blot analyses of microsomal, mitochondrial and cytosolic fractions obtained from transfected HEK293 cells. Fractions corresponding to 40 µg protein were loaded per lane. The last two lanes contain human liver microsomes and cytosol corresponding to 40 µg protein. The membrane was incubated with CYP2W1 antiserum and exposed for two minutes.
**Figure 8**
   5' flanking region deletion constructs in pGL3-Basic.
**Figure 9**
   Comparison of luciferase activity of different promoter lengths. Luciferase assay was performed 24 hours after transfection. Basal luciferase reading (pGL3-Basic) was set at 1.0.
**Figure 10**
   Shorter constructs made from genomic DNA. The region between the transcription initiation site and 78 bp upstream gave a significant increase in luciferase activity.
**Figure 11**
   The region between the transcription initiation site and 78 bp upstream of it, which contains the TATA box, gives a significant increase in luciferase activity.

### Detailed description of the invention

The approach for drug targeting in cancer therapy according to the invention is to utilize a specific form of cytochrome P450 only present in the transformed tissue being able to activate prodrugs converted to cytotoxic products. The present inventors have identified a novel human cytochrome P450 form, CYP2W1, mainly expressed in tumour cells. This finding represents a major step of importance for the development of new potential agents effective in cancer therapy. Thus, the present description discloses the use of the cytochrome P450 enzyme CYP2W1 and genetic variants thereof as a drug target in cancer therapy. CYP2W1 was found by the inventors to be selectively expressed in tumour tissues such as lung carcinoma, colon carcinoma and ovarian carcinoma. A common problem in cancer therapy is the unselective toxicity exerted by cytotoxic drugs against cancer cells and their healthy counterparts. The present description shows the potential to improve drug targeting to tumour cells solely by administering substances that can be metabolically activated by CYP2W1 to cytotoxic agents. Accordingly, the description provides a method for screening for such substances, substrates for CYP2W1, which can be modified by the enzyme to obtain a cytotoxic metabolite. This method comprises the steps:
1. Bringing a candidate prodrug into contact with CYP2W1 or a cell expressing CYP2W1.
2. Allowing CYP2W1 to act on the candidate prodrug to produce a candidate drug.
3. Analysing the candidate drug for cytotoxic/anti-cancer properties.
The candidate prodrug is preferably a non-toxic compound suitable for conventional pharmaceutical administration.
In another preferred method of screening for cytotoxic agents, anticancer agents, or other substrates, activated by CYP2W1, the enzyme is expressed using for example yeast as expression system. Other expression systems like bacterial and mammalian expression system could also be used as enzyme source. The obtained microsomes are incubated with a fluorescent substrate that is metabolised by CYP2W1. Inhibition of that reaction is then used for drug screening with different substrates. Candidate substrates to be tested, but not restricted to, could be drugs with moieties that are known to be metabolised by other members of the CYP2 family. The substrate that shows the best inhibition can be modified in order to obtain a cytotoxic metabolite. Thus the invention relates to a screening assay using CYP2W1 as a drug target.

One aspect of the invention relates to targeting of drugs and prodrugs to cancer cells, preferably to lung, colon or ovarian tumour cells. Since cytochromes P450 are partially expressed on the cell surface, drugs and/or prodrugs can also be targeted to cancer cells by the coupling of them to ligands which bind specifically to CYP2W1. In this aspect, "prodrug" means a substance which will be converted to a cytotoxic and/or anti-cancer drug upon binding to a cell expressing CYP2W1 on its surface. The drug to be administered in this way could be any anticancer/cytotoxic drug known in the art. The prodrug could be a prodrug identified by the screening method described above or any other compound which is activated to a cytotoxic or anti-cancer agent by CYP2W 1. The ligand to which the drug/prodrug should be coupled could be any molecule that binds specifically to CYP2W1 bound on the cell surface, i.e. does not show any substantial specific binding affinity towards any other endogenous or exogenous substances. The ligand could be an antibody, preferably a monoclonal antibody or a binding fragment thereof, which can be prepared by standard techniques. Pharmaceutical compositions comprising the drug/prodrug-ligand compound could be prepared by techniques known in the art.

Yet another aspect of the description relates to the combinatorial use of a prodrug to be metabolised, and a substance that can induce the expression of CYP2W1 in the tumour cells, thus resulting in higher concentrations of the cytotoxic metabolite at the site of the tumour. In a further aspect, the description relates to a method of providing therapeutic agents for cancer therapy, comprising screening for such agents by using CYP2W1 as a drug target. The therapeutic agents shall modulate the activity of CYP2W1, such as induce or increase the activity thereof. This method comprises the steps:
1. Bringing a candidate agent into contact with a cell capable of expressing CYP2W1 or a reporter gene operably linked to the regulatory sequences of CYP2W1.
2. Measuring changes in expression and/or activity of CYP2W1
In order to screen for agents inducing the expression of CYP2W1 different cell lines can be used. One example of possible cell line is the human hepatoma cell line HepG2, which is constitutively expressing CYP2W1. The agent to be tested will be administered to the supplement medium and the induction of the CYP2W1 expression can be tested on the RNA, protein and/or enzymatic activity levels. Candidate agents to be tested, but not restricted to, could be known inducers of cytochrome P450s belonging to the CYP2 and CYP3 family, including those being efficient substrates for the nuclear receptors PXR, LXR, FXR and CAR.

In a yet further aspect, the description relates to a method of treating cancer, comprising giving a patient in need thereof a therapeutically effective amount of a substance activated by the enzyme CYP2W 1 and/or inducing the enzyme CYP2W.
For example, the substance may be activated to an anticancer agent or a cytotoxic substance.

The invention also relates to the use of the CYP2W1 promoter (SEQ ID NO: 10) in gene therapy. This aspect includes the use of the promoter in the manufacture of a medicament, preferably a medicament for use in cancer therapy, even more preferably for use in treatment of lung tumours, colon tumours or ovarian tumours. This aspect also includes the use of DNA-molecules containing the CYP2W1 promoter in pharmaceutical compositions, such DNA-molecules could be, for example, vectors for delivery of the promoter to the patient in need thereof.

In an even further aspect the description relates to the use of the CYP2W1 promoter in screening for modulators, such as inducers or enhancers, of CYP2W1. The promoter can be linked to a variety of reporter systems known in the art in order to study the effects of candidate modulators.

### Experimental

The present invention will be described in more detail below in an experimental section.

### Materials and Methods

### Bioinformatics

The BLASTN search algorithm was used to search the Celera sequence databases (http://www.celera.com) for sequences related to the previously reported CYP2W1 partial cDNA sequence (GenBank Accession No. AK000366). The amino acid sequence of CYP2W1 was compared with the amino acid sequences of other human P450s using multiple sequence alignment. An unrooted phylogenetic tree was calculated using ClustalW 1.8 (Thompson et al., 1997) and visualized using the program unrooted (http://pbil.univ-lyon1.fr/software/umooted.html).

### RNA Isolation and 1^{st} strand cDNA Synthesis

Total RNA was isolated from cultured HepG2 and B16A2 cells using the TRIZOLE Reagent (Invitrogen, Rockville, MD) based on the acid guanidine thiocyanate phenol chloroform extraction method, whereas total RNA from HEK293 and HeLa cells was isolated using the RNeasy Mini Kit (Qiagen, Hilden, Germany) Reverse transcriptase was performed using MMLV Reverse Transcriptase (Invitrogen, Rockville, MD) according to the manufacturer's instructions.

### Amplification and Sequencing

cDNA from HepG2 cells was used as a template in PCR reactions with CYP2W1 specific primers. The enzyme used for amplification was Taq DNA Polymerase (ABgene, Epsom, UK). The PCR products generated were sequenced using the ABI PRISM BigDye Terminator Cycle Sequencing Ready Reaction kit (Applied Biosystems, Foster City, CA), according to the manufacturers instructions, and analyzed on an ABI PRISM 377 DNA Sequencer (Applied Biosystems, Foster City, CA).

### Multiple Tissue cDNA Panels

cDNA from Human Multiple Tissue cDNA (MTC) Panel I and II, Human Digestive System MTC Panel, Human Fetal MTC Panel and Human Tumour MTC Panel (Clontech, Palo Alto, CA) were used as templates in a PCR-reaction using the CYP2W1 specific primer pair, 5'-GAGGTGGAGGCATCTTCTTCTCATCTG-3'/5'-CTGGATCAGGGCGTCCACATAGCTG-3'. A reaction without cDNA was run as a negative control in each experiment. The PCR amplification was performed in the presence of 1 mM MgCl₂, 0.2 mM of each dNTP, 0.4 µM of each primer, 0.625 U Taq DNA Polymerase (ABgene, Epsom, UK), 1x Reaction Buffer IV (75 mM Tris-HCl pH 8.8 at 25°C, 20 mM (NH₄)₂SO₄, 0.01% (v/v) Tween 20) and 0.5 ng cDNA. The PCR reaction was performed by an initial denaturation step at 94°C for 3 min, after which the amplification was carried out for the indicated number of cycles, with denaturation at 94°C for 45s, annealing at 60°C for 30s, and extension at 72°C for 1 min and 30s, and a final extension step of 72°C for 10 min. HepG2 cDNA was used as a positive control, and the quality of the cDNA in the MTC panels was ensured by using the G3PDH control primers, supplied by the manufacturer. Amplification was performed under the same conditions as described above. The PCR products were separated and detected on a 1.2% ethidium-bromide stained agarose gel. The PCR product generated was sequenced and analyzed, as described above, in order to ensure specificity of the reaction.

### Real-time quantitative PCR detection

CYP2W1 mRNA expression was quantified in Human Tumour MTC Panel using the Smart Cycler® System (Cepheid, Sunnyvale, CA). The quantitative real-time PCR assay was carried out using the CYP2W1 gene specific primer pair, 5'-AGCTATGTGGACGCCCTGATCCA-3'/5'-ACGCGGTCTAGCTCCTCCTGCAC-3', and SYBR®Green PCR Master Mix (Applied Biosystems, Foster City, CA). The quantification was performed using 0.5 ng of each cDNA sample. To avoid detection of any possible contaminating genoinic DNA the primer pair was designed so the resulting product spans exon junctions. Standard curves were constructed with the use of serial 10-fold dilutions ranging from 10⁻² fmol/µl to 10⁻⁹ fmol/µl of an accurately determined concentration of a CYP2W1 full-length cDNA fragment. The data was analyzed using the Smart Cycler® Software (Cepheid, Sunnyvale, CA).

### Analysis of mRNA by Northern and Dot Blot

A Human Multiple Tissue Expression (MTE) Array (Clontech, Palo Alto, CA) and a Multiple Tissue Northern (MTN) Blot (Clontech, Palo Alto, CA) were hybridized with a ³²P-labeled CYP2W1 probe, corresponding to nucleotides 329-986, which was generated by PCR and labeled using the Radprime DNA labelling system (Invitrogen, Rockville, MD). Hybridization was performed according to the manufacturer's instructions.
The ³²P-labeled CYP2W1 probe was also hybridized to total RNA from HepG2, B16A2, HEK293 and HeLa cells and total RNA from human liver (provided by Anna Westlind). In particular, 20 µg RNA per lane was loaded on a 1.2% agarose/formaldehyde denaturing gel and subjected to electrophoresis using standard procedures (Struhl, 1993). The RNA was transferred to a Hybond-N+ filter (Amersham Pharmacia Biotech, Uppsala, Sweden) and hybridized using ExpressHyb Hybridization Solution (Clontech, Palo Alto, CA). Prehybridization was carried out at 65°C for 30 minutes, and hybridisation was carried out at the same temperature over night. After hybridisation all three blots were exposed to an imaging plate and analysed by a Bats-1800II phosphoimager using the Image Gauge software (Fujifilm, Samford, CT).

### Cell Culture Conditions

HepG2 and HEK293 cells were cultured in Minimum Essential Medium supplemented with 10% fetal bovine serum, 1mM sodium pyruvate, 1% non-essential amino acids, 100 IU/ml penicillin and 100 µg/ml streptomycin. HeLa cells were cultured in Dulbecco's Modified Eagle Medium supplemented with 10% fetal bovine serum, 1% non-essential amino acids, 100 IU/ml penicillin and 100 µg/ml streptomycin. B16A2 cells were cultured in William's E Medium supplemented with 5% fetal bovine serum, 0.6 µM hydrocortisone, 0.25 µg/ml insulin, 100 IU/ml penicillin and 100 µg/ml streptomycin. All cells were cultured in humidified 5% CO₂ at 37°C. All cell culture media and supplements were obtained from Invitrogen, Rockville, MD.

### CYP2W1 Antiserum

An 15 amino acid long peptide, TMRPRAQALCAVPRP, corresponding to the C-terminus of CYP2W1 was synthesized and coupled to keyhold limpet hemocyanin (Neosystems, Strasbourg, France). Immunization of two rabbits was carried out with injections at day 0, 14, 27 and 56. Bleedings were performed before the first injection to obtain pre-immune serum, and after 39, 67 and 96 days to obtain the CYP2W1-specific antiserum.

### Preparation of Total Cell Extracts and Western Blot

The human cell lines HepG2, HEK293, HeLa and B16A2 were analysed for CYP2W1 expression using western blot. Cells were washed twice, scraped into ice-cold PBS and pelleted by centrifugation at 17,320x g. The pelleted cells were resuspended in a phosphate buffer, containing 50 mM NaPO₄ pH 7.4, 0.1mM EDTA and 10% glycerol, and lysed by sonication. After centrifugation at 17,320x g, supernatants were collected and protein concentration was determined according to Lowry. 40 µg total cell extract as well as human liver microsomes and cytosol, were subjected to SDS-PAGE using a 10% gel. The proteins were subsequently transferred to a Hybond-C extra membrane (Amersham Pharmacia Biotech, Uppsala, Sweden). After transfer, the membrane was blocked in TBS containing 0.05% (v/v) Tween 20 and 5% fat-free milk, and incubated with a 1:5000 dilution of the CYP2W1 antiserum. This was followed by an incubation with 1:2000 diluted horseradish peroxidase-conjugated anti-rabbit immunoglobulins and detection using SuperSignal West Pico Chemiluminiscent Substrate (Pierce, Rockford, IL). Blocking experiments were performed using antiserum pre-incubated with peptide. The CYP2W1 antiserum was incubated together with the peptide used for immunisation, at a concentration of 3 µg peptide per µl antiserum. Incubation was performed at room temperature for 2 hours under gentle agitation. After incubation, the antiserum was diluted and immunoblotting was carried out, as described above.

### Construction of CYP2W1 Expression Plasmids

A full-length cDNA clone of CYP2W1 was amplified from cDNA derived from HepG2 cells using Elongase Enzyme Mix (Invitrogen, Rockville, MD). The primer pair used was 5'-GACAGATCTATGGCCCTGCTGCTCTTG-3'/5'-GACTCTAGACTAGGGCCTGGGCACCGCA-3'. Restriction sites for *Bg*/II and *Xba*I (underlined) were introduced by the primers. The PCR reaction contained 0.2 mM of each dNTP, 0.4 µM of each primer, 2.5 U Elongase Enzyme Mix (Clontech, Palo Alto, CA), and a Buffer A/Buffer B ratio corresponding to 1.7 mM MgCl₂ (5x Buffer A containing 300 mM Tris-SO₄ pH 9.1, 90 mM (NH₄)₂SO₄, and 5 mM MgSO₄; 5x Buffer B containing 300 mM Tris-SO₄ pH 9.1, 90 mM (NH₄)₂SO₄, and 10 mM MgSO₄). The conditions for the reaction were as follows, a denaturation step at 94°C for 1 min followed by 70°C for 1 min when Buffer A/Buffer B and Elongase Enzyme Mix were added as a hot start. Before amplification an additional denaturation step at 94°C for 1 min was performed. The amplification was carried out with denaturation at 94°C for 30s, annealing at 52°C for 30s, and extension at 68°C for 2 min for 35 cycles, and a final extension step of 68°C for 7 min. The obtained PCR product was digested with *Bg*/II and *Xba*I (Invitrogen, Rockville, MD) and ligated into the expression vector pCMV4, cut with the same restriction enzymes as the PCR product. The sequence inserted was verified by DNA sequencing.

### Heterologous Expression in Mammalian Cells

The HEK293 cell line was used for expression of the CYP2W1 protein. The cells were transfected with 30 µg pCMV4-2W1 plasmid per 15 cm cell culture dish using Lipofectamine 2000 (Invitrogen, Rockville, MD) according to the manufacturers instructions. Cells transfected with only pCMV4 was used as a negative control. The cells were harvested 66 h after transfection and homogenized in 10 mM Tris/HCl pH 7.4 containing 1 mM EDTA, 0.5 M sucrose and Complete protease inhibitor (Roche, Mannheim, Germany). The homogenate was fractionated by centrifugation, and the microsomal fraction was used for estimation of the total P450 content The microsomal, mitochondrial and cytosolic fractions were subjected to western blot for detection of the CYP2W1 protein, as described above.

### Identification of promoter

Varying lengths of the 5' flanking region of the *CYP2W1* gene were subcloned into the reporter vector, pGL3-Basic. These were transfected into the hepatoma cell line HepG2 (Lipofectamine 2000, Invitrogen, and Tfx-20 Reagent, Promega) 24 hours prior to luciferase assay (Dual-Luciferase Reporter Assay System, Promega). The region that gave the highest luciferase activity was further analyzed by preparing constructs of shorter intervals. A transcription factor search was the basis for making these constructs.

### Results

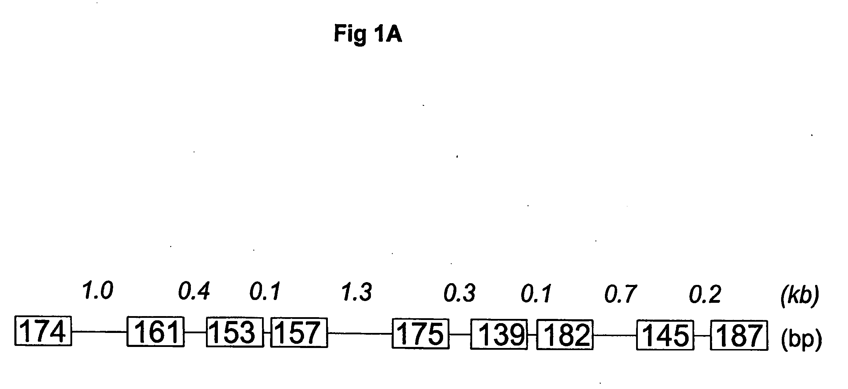

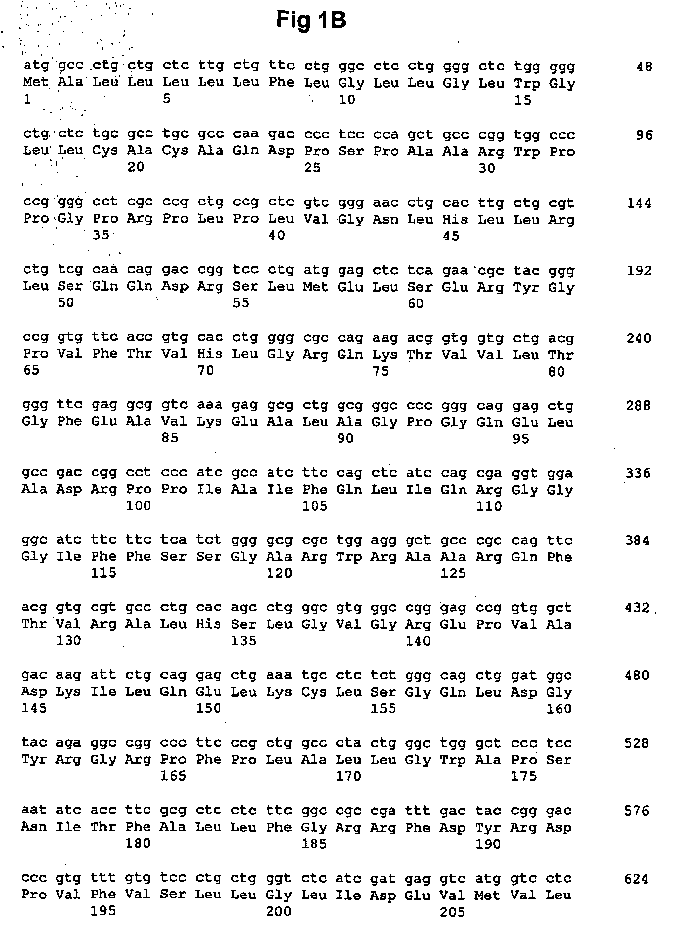

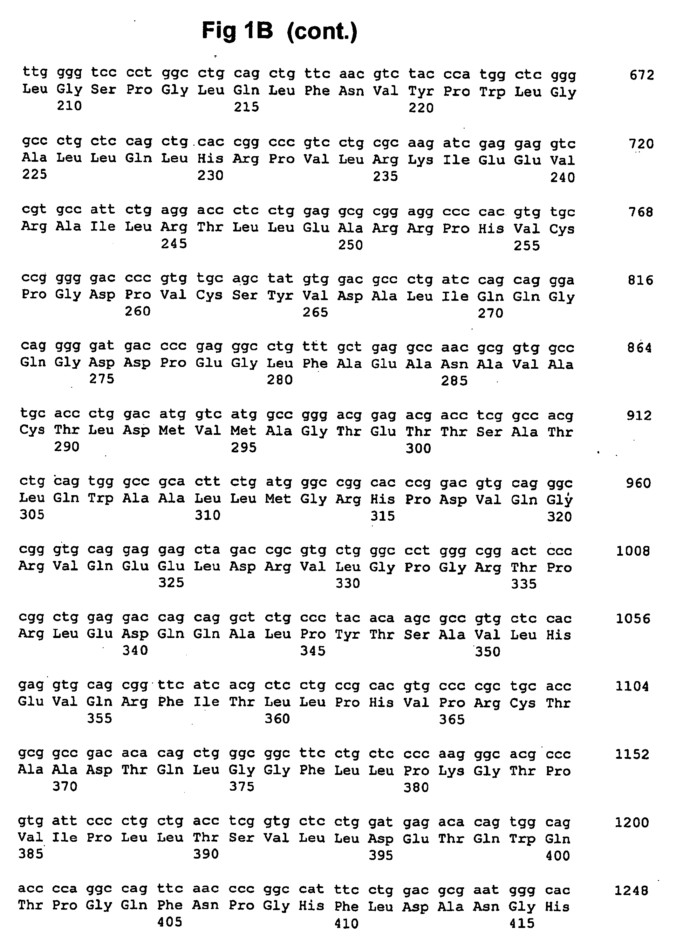

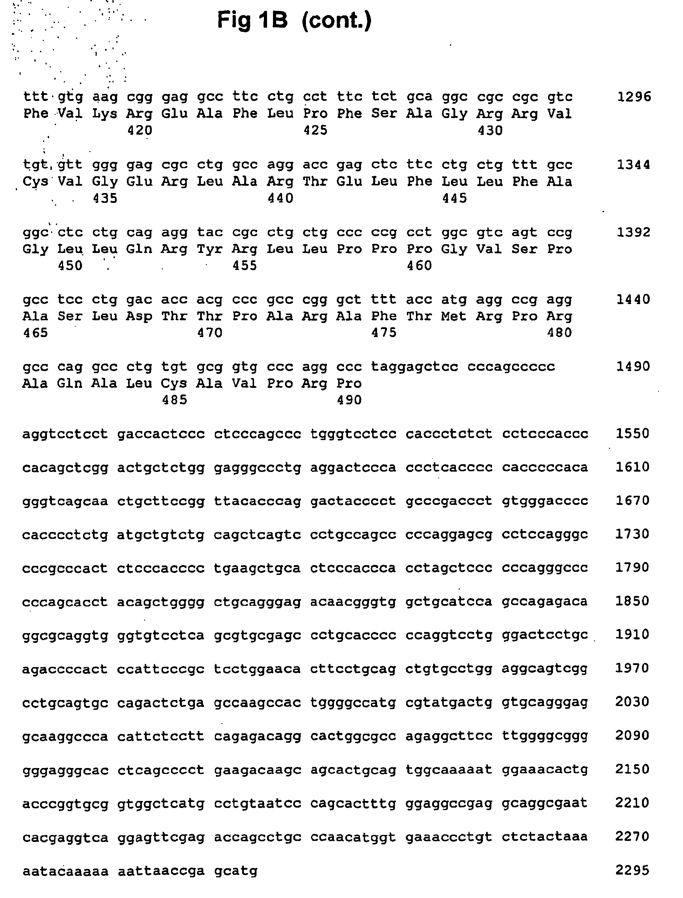

A number of genomic clones related to CYP2W1 were identified and aligned together in order to obtain the *CYP2W1* gene sequence. A Celera transcript, hCT1786066, was also identified which contained the 3' untranslated region of the cDNA. The *CYP2W1* gene was more than 5.5 kb long and showed the typical family 2 gene structure with nine exons (Fig. 1A). The obtained gene sequence was used when designing primers for the amplification and sequencing of the entire HepG2 CYP2W1 cDNA. Sequencing resulted in an open reading frame of 1473 nucleotides, which encodes a 490 amino acid long polypeptide. The nucleotide and predicted amino acid sequence of CYP2W1 are shown in figure 1B. CYP2W1 was found to contain some typical structural features associated with P450s, including a hydrophobic NH₂-terminal, the proline-rich region and the conserved cysteine, which is the fifth heme iron ligand. The CYP2W1 cDNA sequence described here differs extensively from the previously reported partial sequence (GenBank Accession No. AK000366) with respect to the N- and C-terminal exons.

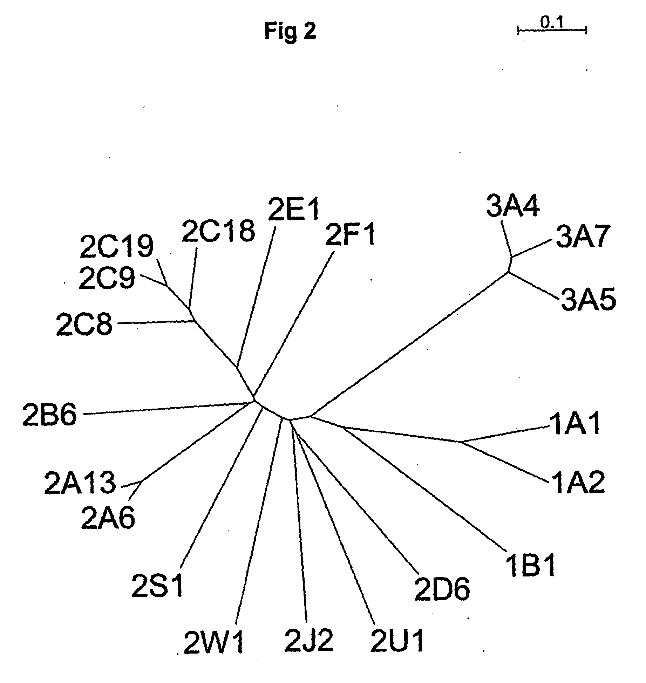

An unrooteed phylogenetic tree of CYP2W1 and other human P450s is shown in figure 2. It shows that CYP2W1 align together with the other family 2 members, but it does not seem to be closely related to any of them. The enzymes within family 2 with highest identity to CYP2W1 are CYP2D6 (42%) and CYP2S1 (40%).

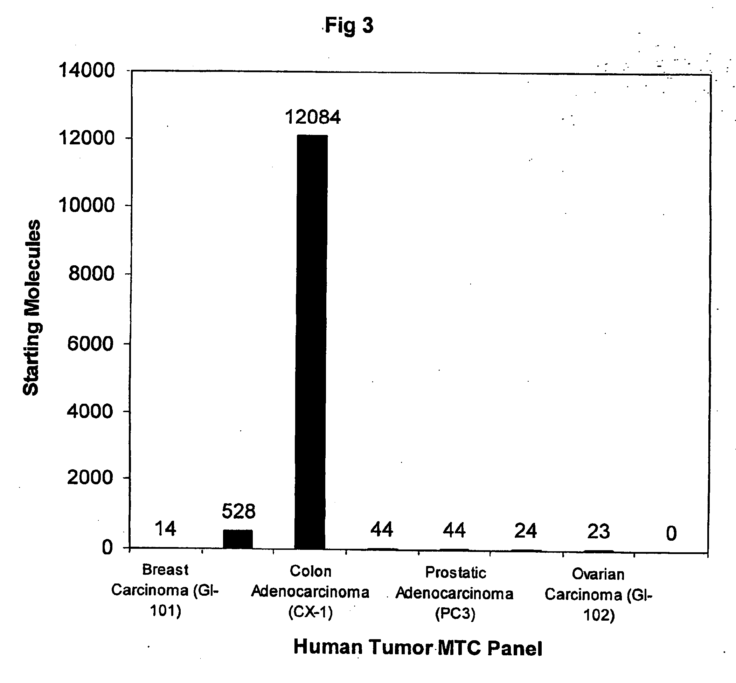

The tissue-specific CYP2W1 mRNA distribution was analysed by using MTC panels by PCR. The cDNA samples in the MTC panels were pooled from several individuals and normalized with at least four different housekeeping genes. As shown in Fig 3 and Table 1 the tissues that showed the highest expression of CYP2W1 mRNA were the tumour tissues lung carcinoma and colon adenocarcinoma. Moderate expression was seen in for example fetal lung, but the expression levels seen in the majority of normal adult and fetal tissues were low or absent. A rough calculation about the difference in expression between the tumour cells having highest CYP2W1 mRNA and normal tissue reveals a 10,000-100,000 fold difference.

**Table 1**

| Tissue specific expression of CYP2W1 revealed by PCR analyses using gene specific primers and different Human Multiple Tissue cDNA (MTC) Panels. The number of cycles required to obtain a visible product band on an ethidium-bromide stained gel is given in the column. The abbreviation nd stands for not detected, which means that no product band could be seen after 40 cycles of amplification. | | |
|---|---|---|
| **Panel** | **Tissue** | **Cycles for Detection** |
| **Human MTC Panel I** | Brain | nd |
| | Heart | nd |
| | Kidney | 36-40 |
| | Liver | 36-40 |
| | Lung | nd |
| | Pancreas | 31-35 |
| | Placenta | 36-40 |
| | Skeletal Muscle | 36-40 |
| **Human MTC Panel II** | Colon | 31-35 |
| | Ovary | 36-40 |
| | Peripheral Blood Leukocytes | nd |
| | Prostate | 31-35 |
| | Small Intestine | nd |
| | Spleen | nd |
| | Testis | nd |
| | Thymus | nd |
| **Human Digestive System** | Cecum | 36-40 |
| **MTC Panel** | Colon, Ascending | 36-40 |
| | Colon, Descending | 36-40 |
| | Colon, Transverse | 36-40 |
| | Duodenum | 31-35 |
| | Esophagus | 36-40 |
| | Ileocecum | 36-40 |
| | Ileum | 31-35 |
| | Jejunum | 26-30 |
| | Liver | 31-35 |
| | Rectum | 26-30 |
| | Stomach | 31-35 |
| **Human Fetal MTC Panel** | Brain | nd |
| | Heart | nd |
| | Kidney | 31-35 |
| | Liver | 31-35 |
| | Lung | 26-30 |
| | Skeletal Muscle | nd |
| | Spleen | nd |
| | Thymus | nd |
| **Human Tumour MTC Panel** | Breast Carcinoma (GI-101) | nd |
| | Lung Carcinoma (LX-1) | 21-25 |
| | Colon Adenocarcinoma (CX-1) | 21-25 |
| | Lung (GI-117) | 36-40 |
| | Prostatic Adenocarcinoma (PC3) | 31-35 |
| | Colon Adenocarcinoma (GI-112) | 36-40 |
| | Ovarian Carcinoma (GI-102) | 26-30 |
| | Pancreatic Adenocarcinoma (GI-103) | nd |

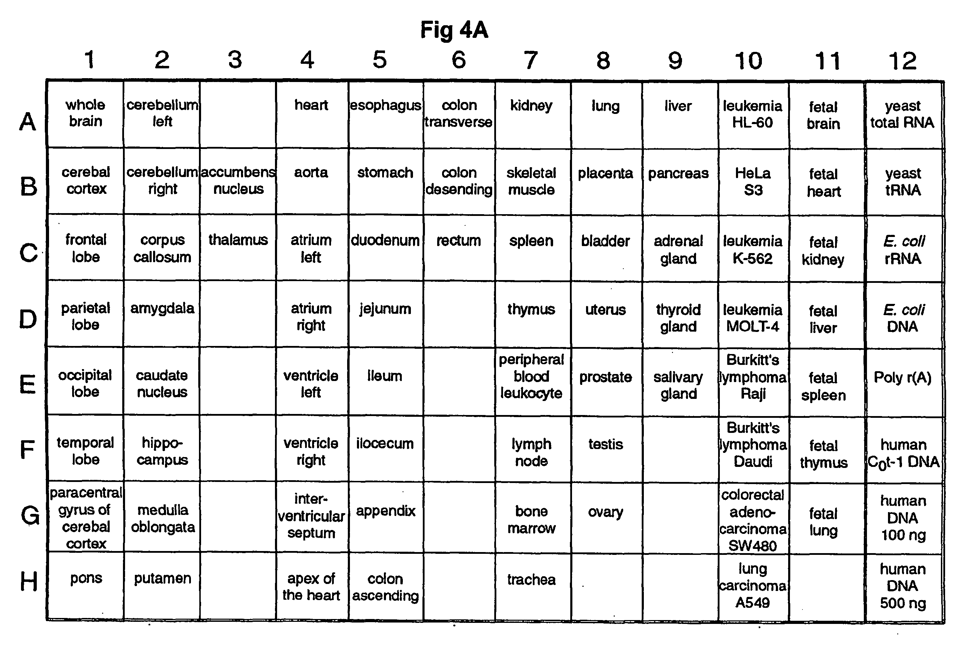

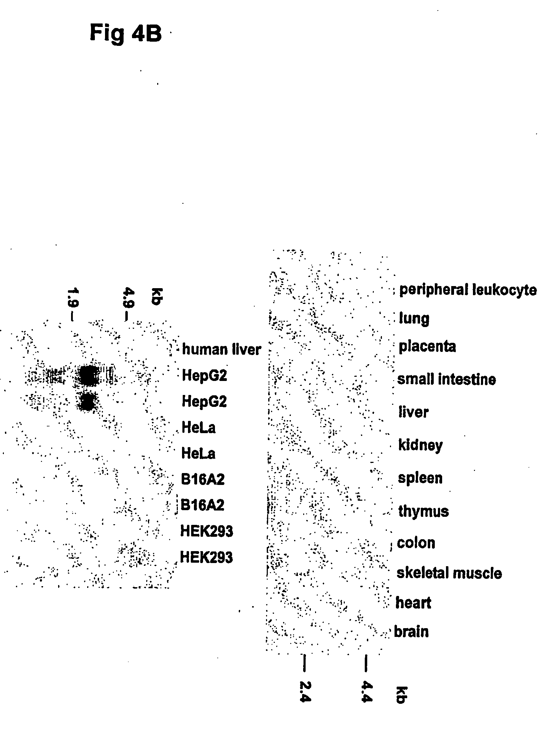

The mRNA distribution was also examined using different mRNA blots, like MTE Array, MTN blot and Northern blotting. The mRNA amounts on the MTE Array have been normalized using eight different housekeeping genes, and the MTN blot has been normalized with respect to β-actin. Using these two methods for mRNA analysis no signals, and hence no expression, could be observed in any of the tissues (Fig. 4A and B). A Northern blot analysis was also performed using RNA from HepG2, B16A2, HEK293 and HeLa cells and human liver. In HepG2 cells a strong signal corresponding to a transcript of approximately 2.3 kb was observed (Fig.4B), which corresponds well to the sequence shown in Figure 1B. No signals could be obtained in the other cell lines or in human liver, which is consistent with the low CYP2W1 mRNA expression seen in liver using the MTC panels.

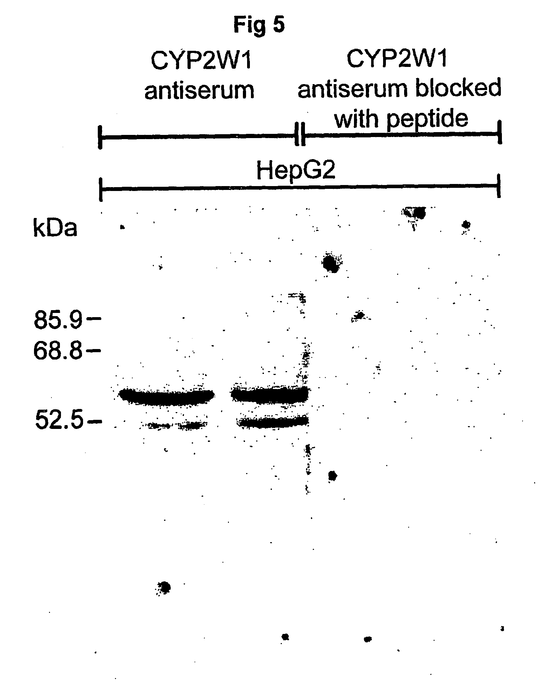

A CYP2W1 antiserum was produced by immunization of rabbits, using the C-terminal sequence of CYP2W1. The cell lines examined for the presence of CYP2W1 using western blotting were HepG2, HEK293, HeLa and B16A2, and in addition, we also determined the content in human liver microsomes and cytosol. The membranes containing HepG2 total cell extract and incubated with CYP2W1 antiserum recognized two bands of approximately 54 and 52 kDa as seen in Figure 5. The upper band is the result of unspecific binding to a cytosolic protein, whereas the size of the lower band corresponds well to the calculated CYP2W1 protein sequence of 490 amino acids. When immunoblotting was made using pre-immune serum (data not shown) or antiserum blocked with the immunizating peptide the previously seen bands were abolished (Fig. 5). No band corresponding to CYP2W1 was seen in human liver or in the other cell lines examined (data not shown). Cross-reactivity with other P450s could be excluded since no band was observed in human liver microsomes, which normally shows high expression of most P450s in families 1-3.

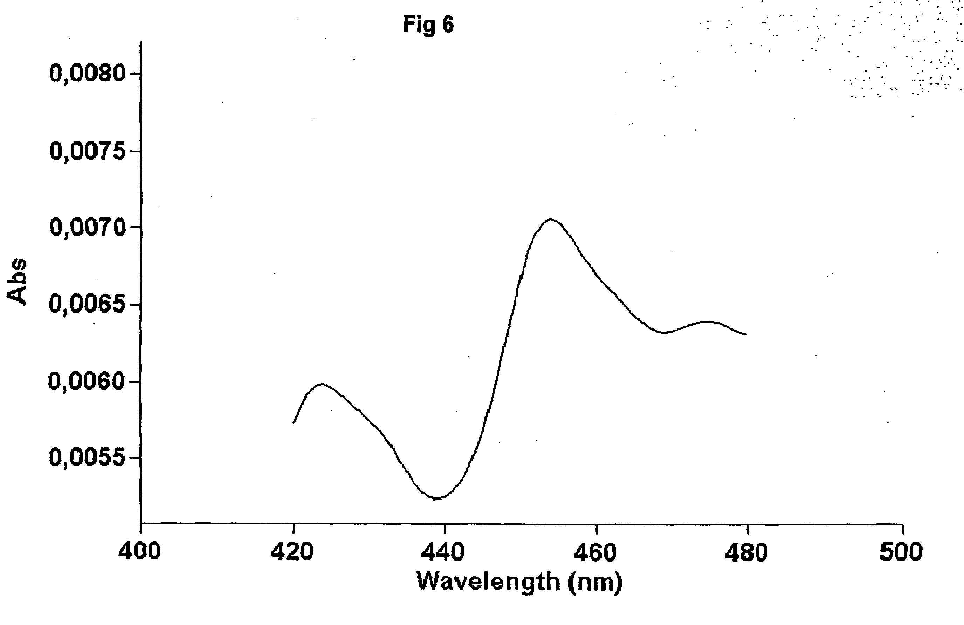

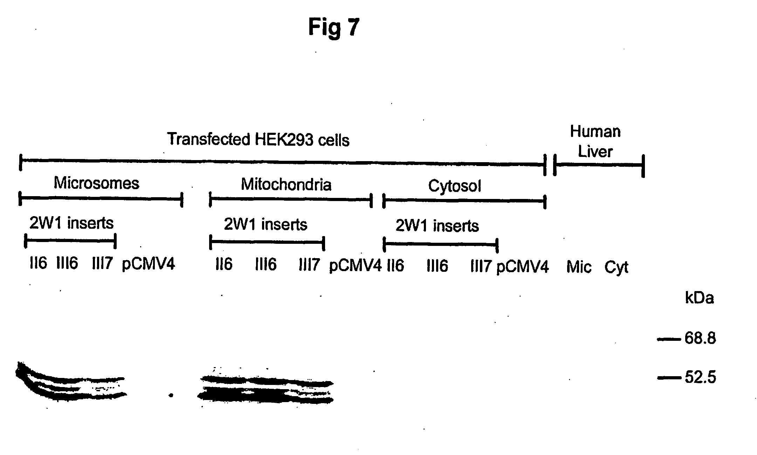

CYP2W1 was heterologously expressed in HEK293 cells using three different pCMV4-2W1 constructs (II6, III6 and III7) and empty pCMV4 vector as a negative control. The microsomal fraction of the transfected cells was used for an estimation of the P450 content by spectral analysis of the reduced CO-bound form (Fig. 6). A peak was observed at approximately 450 nm indicating presence of active P450 enzyme. The amount of P450 obtained was 5 pmol/mg microsomal protein. The microsomal, mitochondrial and cytosolic fractions were subjected to western blotting for detection of the CYP2W1 protein (Fig. 7). A strong band indicating CYP2W1 were seen in the microsomal and mitochondrial fractions transfected with all pCMV4-2W1 constructs whereas no bands could be seen in the negative control.

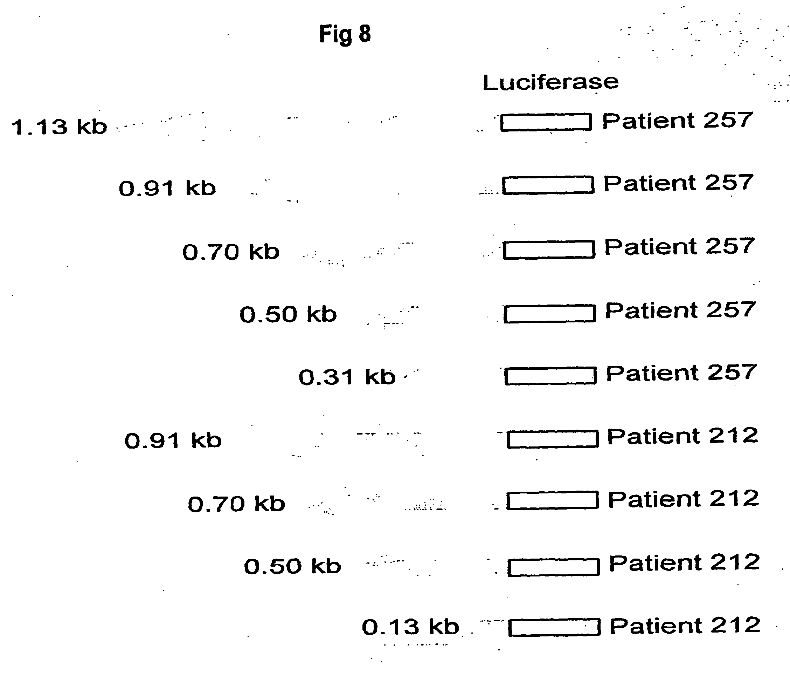

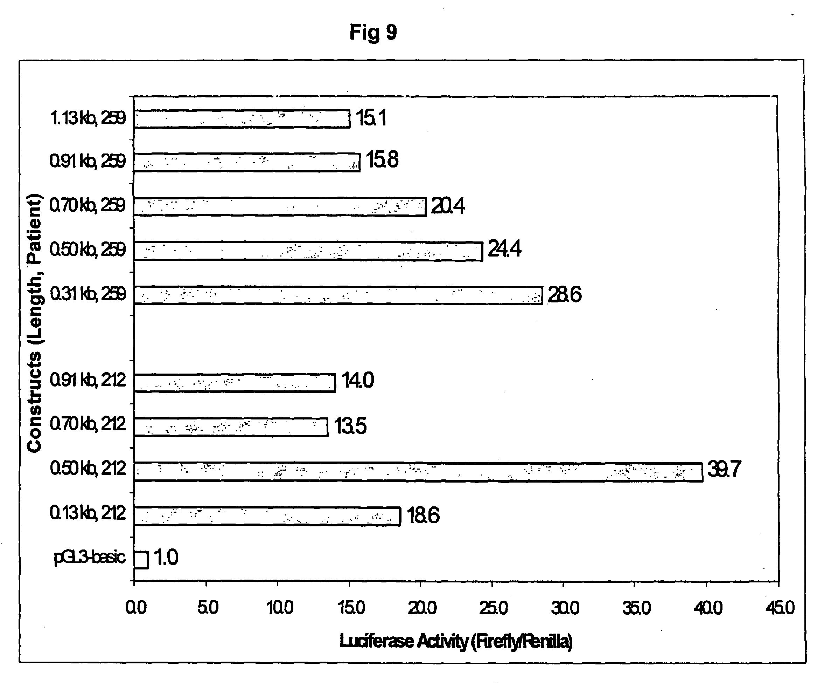

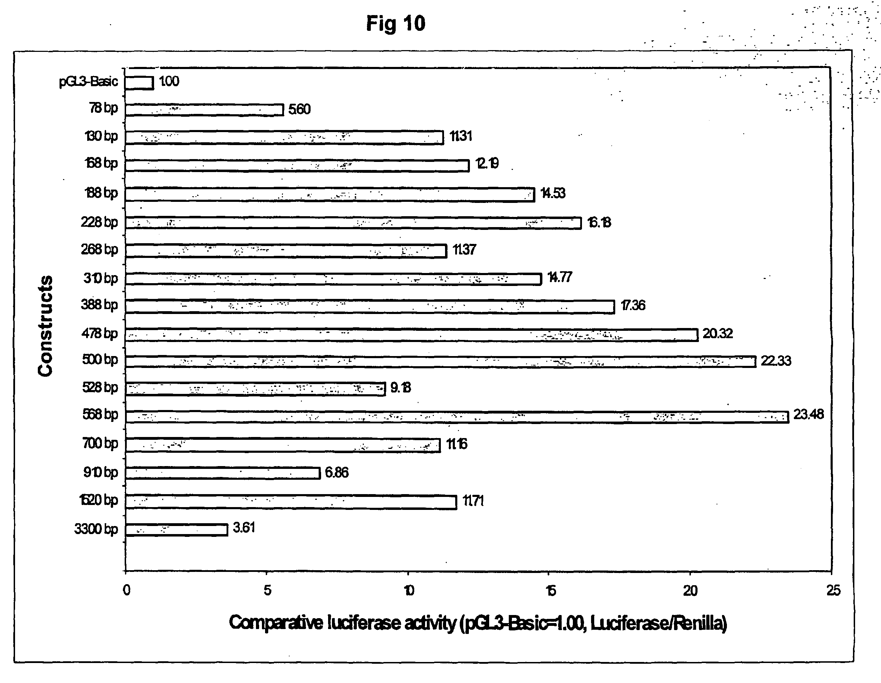

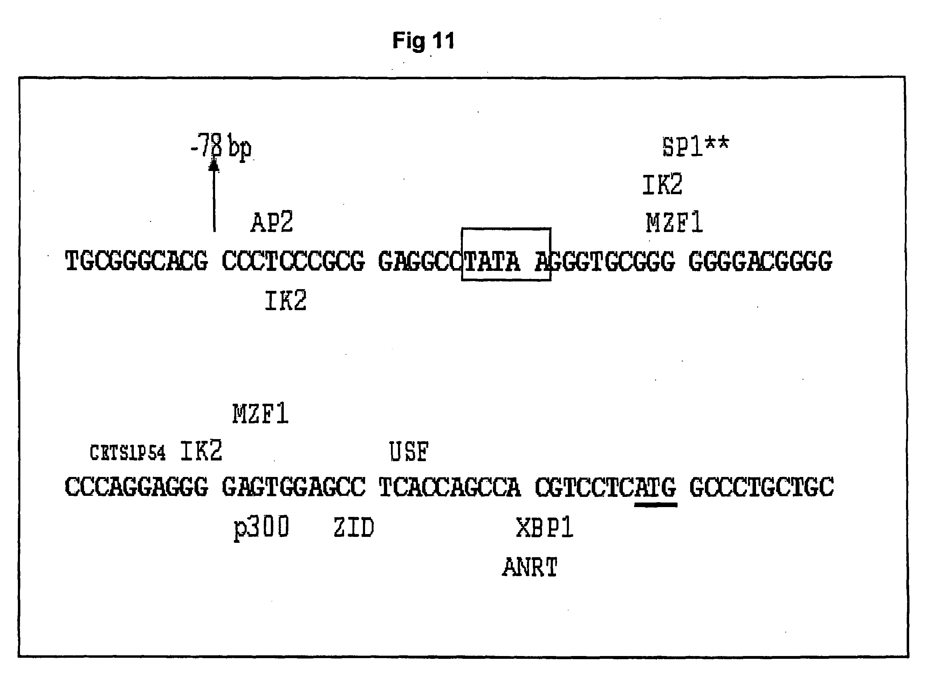

Different parts of the 5'-flanking region of the CYP2W1 gene were subcloned to a reporter vector pGL3-Basic which bears the luciferase gene. Hepatoma cells HepG2 were used for the transfection experiments. Initial analysis of different lengths of the *CYP2W1* promoter region (Figure 8) suggests that the region between 130 bp and 500 bp upstream of the transcription initiation site (Figure 9, Subject 212) may possess a binding site for a transcription factor that can maximally drive the expression of CYP2W1. Shorter fragments from this region were analyzed (Figure 10). The shortest fragment included in the experiment gave a significant increase in luciferase activity (Figure 10) and this region contains the TATA box where RNA polymerase II binds and initiates transcription (Figure 11). Several transcriptional factors and activators are expected to bind to this region and may also affect the level of expression of CYP2W1.

It is concluded that the region between -1 and -130 bp, SEQ ID NO: 10, constitutes a very strong promoter for expression of CYP2W1 in the hepatoma cells.

### Discussion

Alignment of genomic clones allowed the construction of the gene structure of CYP2W1. Based upon this it was possible to make a full length cDNA construct using mRNA from HepG2 cells which revealed a P450 enzyme with expected length and sequence motifs. Semiquantitative analysis for the amount of mRNA expressed in human MTC panels revealed the highest expression in a lung, a colon and an ovarian tumour, whereas in general only small mRNA expression was seen in preparations from adult tissues. Based upon the number of cycles duringPCR necessary to amplify the product a rough calculation reveals 10,000-100,000 fold higher CYP2W1 mRNA expressed in these tumour cells than in non-transformed cells. Further quantitative estimations are now done using Northern blot filters from different human tumours. The inventors detected significant mRNA expression in HepG2 cells whereas, by contrast, they could not see any significant expression as determined by Northern blotting in different tissues from adult human.

The inventors successfully obtained a specific antiserum to the protein. Western blotting experiments revealed that no significant expression was seen in human liver, but that high expression was seen in the transformed cell line HepG2. This is promising in order to obtain specific Western blotting analyses for detection of the enzyme in different tumours. In HEK293 cells heterologously expressed with CYP2W1 the expression of three bands was seen with slightly different molecular weights. The significance of this observation is currently evaluated.

Large cultivation of HEK293 cells transfected with pCMV-CYP2W1 cDNA allowed the preparation of microsomes useful for detection of spectrally identifiable CYP2W1. The results showed a typical P450 spectrum indicating proper folding of the enzyme being catalytically active.

The transformed cells line HepG2 has not been described to express any known form of microsomal cytochrome P450 at significant levels. Unexpectedly, the present inventors found relatively high CYP2W1 expression as revealed by the Western blotting experiments in HepG2 cells. The background for this is unknown but current work aims at identifying the transcription factors and cis acting elements required for this expression. In addition, the semiquantitative determination of CYP2W1 in some different human tumours revealed an exceptionally high expression.

The present inventors propose use of the enzyme CYP2W1 as a drug target for the development of cytotoxic drugs aimed for cancer therapy. Previously the extrahepatic CYP1B1 form has been shown to be more abundantly expressed in tumours than in non-transformed tissue whereas CYP2W1 appears to represent a much more important example of tumour specific cytochrome P450 expression.

### References

Ingelman-Sundberg M., Cytochrome P450 enzymes,. Naunyn-Schmiedeberg's archives of pharmacology, 2003, in press.
Jounaidi Y, Waxman DJ. Frequent, , moderate-dose cyclophosphamide administration improves the efficacy of cytochrome P-450/cytochrome P-450 reductase-based cancer gene therapy. Cancer Res. 2001 ;61:4437-44.
Thompson JD, Gibson TJ, Plewniak F, Jeanmougin F, Higgins DG. The CLUSTAL_X windows interface: flexible strategies for multiple sequence alignment aided by quality analysis tools. Nucleic Acids Res. 1997 ; 25:4876-82.
Waxman DJ, Chen L, Hecht JE, Jounaidi Y. Cytochrome P450-based cancer gene therapy: recent advances and future prospects. Drug Metab Rev. 1999;31:503-22.

## Claims

1. A compound comprising one part conferring cytotoxic and/or anti-cancer effects to the compound and one part conferring binding affinity towards CYP2W1 to the compound.

2. A pharmaceutical composition comprising a compound according to claim 1 and pharmaceutically acceptable excipients and/or carriers.

3. An antibody, preferably a monoclonal antibody, binding specifically to CYP2W1.

4. DNA-molecule with nucleotide sequence according to SEQ ID NO: 10.

5. Use of a DNA-molecule according to claim 4 in the manufacture of a medicament.

## Patentansprüche

1. Verbindung, umfassend einen Teil, der der Verbindung zytotoxische und/oder anti-Krebseffekte verleiht, und einen Teil, der der Verbindung Bindungsaffinitität für CYP2W1 verleiht.

2. Pharmazeutische Zusammensetzung umfassend eine Verbindung gemäß Anspruch 1 und pharmazeutisch verträgliche Excipienten und/oder Träger.

3. Antikörper, vorzugsweise monoclonaler Antikörper, der spezifisch CYP2W1 bindet.

4. DNA-Molekül mit Nucleotidsequenz gemäß SEQ ID NO: 10.

5. Verwendung eines DNA-Moleküls nach Anspruch 4 in der Herstellung eines Medikaments.

## Revendications

1. Composé comprenant une partie conférant des effets cytotoxiques et/ou anticancéreux au composé et une partie conférant une affinité de liaison pour CYP2W1 au composé.

2. Composition pharmaceutique comprenant un composé selon la revendication 1 et des excipients et/ou supports pharmaceutiquement acceptables.

3. Anticorps, de préférence un anticorps monoclonal, se liant spécifiquement à CYP2W1.

4. Molécule d'ADN ayant la séquence nucléotidique selon la SEQ ID NO: 10.

5. Utilisation d'une molécule d'ADN selon la revendication 4 dans la fabrication d'un médicament.


### SEQUENCE LISTING

<110> Karolinska Innovations AB
   Ingelman-Sundberg, Magnus
   Karlgren, Maria
   Gomez, Alvin
<120> Drug target in cancer therapy
<130> P05980PC00/HAM/em
<150> SE0203137-5
   <151> 2002-10-24
<150> us 60/420,787
   <151> 2002-10-24
<160> 10
<170> PatentIn version 3.1
<210> 1
   <211> 27
   <212> DNA
   <213> Homo sapiens
<220>
   <221> Primer
   <222> (1)..(27)
   <223>
<400> 1
   gaggtggagg catcttcttc tcatctg 27
<210> 2
   <211> 25
   <212> DNA
   <213> Homo sapiens
<220>
   <221> primer
   <222> (1)..(25)
   <223>
<400> 2
   ctggatcagg gcgtccacat agctg 25
<210> 3
   <211> 23
   <212> DNA
   <213> homo sapiens
<220>
   <221> primer
   <222> (1)..(23)
   <223>
<400> 3
   agctatgtgg acgccctgat cca 23
<210> 4
   <211> 23
   <212> DNA
   <213> homo sapiens
<220>
   <221> primer
   <222> (1)..(23)
   <223>
<400> 4
   acgcggtcta gctcctcctg cac 23
<210> 5
   <211> 15
   <212> PRT
   <213> homo sapiens
<220>
   <221> peptide
   <222> (1)..(15)
   <223>
<400> 5
   Thr Met Arg Pro Arg Ala Gln Ala Leu Cys Ala Val Pro Arg Pro
   1 5 10 15
<210> 6
   <211> 27
   <212> DNA
   <213> homo sapiens
<220>
   <221> primer
   <222> (1)..(27)
   <223>
<400> 6
   gacagatcta tggccctgct gctcttg 27
<210> 7
   <211> 28
   <212> DNA
   <213> homo sapiens
<220>
   <221> primer
   <222> (1)..(28)
   <223>
<400> 7
   gactctagac tagggcctgg gcaccgca 28
<210> 8
   <211> 490
   <212> PRT
   <213> homo sapiens
<400> 8
   Met Ala Leu Leu Leu Leu Leu Phe Leu Gly Leu Leu Gly Leu Trp Gly
   1               5                   10                  15
   Leu Leu Cys Ala Cys Ala Gln Asp Pro Ser Pro Ala Ala Arg Trp Pro
               20                  25                  30
   Pro Gly Pro Arg Pro Leu Pro Leu Val Gly Asn Leu His Leu Leu Arg
           35                  40                  45
   Leu Ser Gln Gln Asp Arg Ser Leu Met Glu Leu Ser Glu Arg Tyr Gly
       50                  55                  60
   Pro Val Phe Thr Val His Leu Gly Arg Gln Lys Thr Val Val Leu Thr
   65                  70                  75                  80
   Gly Phe Glu Ala Val Lys Glu Ala Leu Ala Gly Pro Gly Gln Glu Leu
                   85                  90                  95
   Ala Asp Arg Pro Pro Ile Ala Ile Phe Gln Leu Ile Gln Arg Gly Gly
               100                 105                 110
   Gly Ile Phe Phe Ser Ser Gly Ala Arg Trp Arg Ala Ala Arg Gln Phe
           115                 120                 125
   Thr Val Arg Ala Leu His Ser Leu Gly Val Gly Arg Glu Pro Val Ala
       130                 135                 140
   Asp Lys Ile Leu Gln Glu Leu Lys Cys Leu Ser Gly Gln Leu Asp Gly
   145                 150                 155                 160
   Tyr Arg Gly Arg Pro Phe Pro Leu Ala Leu Leu Gly Trp Ala Pro Ser
                   165                 170                 175
   Asn Ile Thr Phe Ala Leu Leu Phe Gly Arg Arg Phe Asp Tyr Arg Asp
               180                 185                 190
   Pro Val Phe Val Ser Leu Leu Gly Leu Ile Asp Glu Val Met Val Leu
           195                 200                 205
   Leu Gly Ser Pro Gly Leu Gln Leu Phe Asn Val Tyr Pro Trp Leu Gly
       210                 215                 220
   Ala Leu Leu Gln Leu His Arg Pro Val Leu Arg Lys Ile Glu Glu Val
   225                 230                 235                 240
   Arg Ala Ile Leu Arg Thr Leu Leu Glu Ala Arg Arg Pro His Val Cys
                   245                 250                 255
   Pro Gly Asp Pro Val Cys Ser Tyr Val Asp Ala Leu Ile Gln Gln Gly
               260                 265                 270
   Gln Gly Asp Asp Pro Glu Gly Leu Phe Ala Glu Ala Asn Ala Val Ala
           275                 280                 285
   Cys Thr Leu Asp Met Val Met Ala Gly Thr Glu Thr Thr Ser Ala Thr
       290                 295                 300
   Leu Gln Trp Ala Ala Leu Leu Met Gly Arg His Pro Asp Val Gln Gly
   305                 310                 315                 320
   Arg Val Gln Glu Glu Leu Asp Arg Val Leu Gly Pro Gly Arg Thr Pro
                   325                 330                 335
   Arg Leu Glu Asp Gln Gln Ala Leu Pro Tyr Thr Ser Ala Val Leu His
               340                 345                 350
   Glu Val Gln Arg Phe Ile Thr Leu Leu Pro His Val Pro Arg Cys Thr
           355                 360                 365
   Ala Ala Asp Thr Gln Leu Gly Gly Phe Leu Leu Pro Lys Gly Thr Pro
       370                 375                 380
   Val Ile Pro Leu Leu Thr Ser Val Leu Leu Asp Glu Thr Gln Trp Gln
   385                 390                 395                 400
   Thr Pro Gly Gln Phe Asn Pro Gly His Phe Leu Asp Ala Asn Gly His
                   405                 410                 415
   Phe Val Lys Arg Glu Ala Phe Leu Pro Phe Ser Ala Gly Arg Arg Val
               420                 425                 430
   Cys Val Gly Glu Arg Leu Ala Arg Thr Glu Leu Phe Leu Leu Phe Ala
           435                 440                 445
   Gly Leu Leu Gln Arg Tyr Arg Leu Leu Pro Pro Pro Gly Val Ser Pro
       450                 455                 460
   Ala Ser Leu Asp Thr Thr Pro Ala Arg Ala Phe Thr Met Arg Pro Arg
   465 470 475 480
   Ala Gln Ala Leu Cys Ala Val Pro Arg Pro
   485 490
<210> 9
   <211> 2432
   <212> DNA
   <213> homo sapiens
<220>
   <221> promoter
   <222> (1)..(137)
   <223>
<220>
   <221> exon
   <222> (138)..(1608)
   <223>
<400> 9
   gccctgcaaa ctgctcgttc cacattctcg gggtggtggg gtgggtgggg tgcgggcacg 60
   ccctcccgcg gaggcctata agggtgcggg ggggacgggg cccaggaggg gagtggagcc 120
   tcaccagcca cgtcctc atg gcc ctg ctg ctc ttg ctg ttc ctg ggc ctc 170
   Met Ala Leu Leu Leu Leu Leu Phe Leu Gly Leu
   1 5 10
   ctg ggg ctc tgg ggg ctg ctc tgc gcc tgc gcc caa gac ccc tcc cca 218
   Leu Gly Leu Trp Gly Leu Leu Cys Ala Cys Ala Gln Asp Pro Ser Pro
   15 20 25
   gct gcc cgg tgg ccc ccg ggg cct cgc ccg ctg ccg ctc gtc ggg aac 266
   Ala Ala Arg Trp Pro Pro Gly Pro Arg Pro Leu Pro Leu Val Gly Asn
   30 35 40
   ctg cac ttg ctg cgt ctg tcg caa cag gac cgg tcc ctg atg gag ctc 314
   Leu His Leu Leu Arg Leu Ser Gln Gln Asp Arg Ser Leu Met Glu Leu
   45 50 55
   tca gaa cgc tac ggg ccg gtg ttc acc gtg cac ctg ggg cgc cag aag 362
   Ser Glu Arg Tyr Gly Pro Val Phe Thr Val His Leu Gly Arg Gln Lys
   60 65 70 75
   acg gtg gtg ctg acg ggg ttc gag gcg gtc aaa gag gcg ctg gcg ggc 410
   Thr Val Val Leu Thr Gly Phe Glu Ala Val Lys Glu Ala Leu Ala Gly
   80 85 90
   ccc ggg cag gag ctg gcc gac cgg cct ccc atc gcc atc ttc cag ctc 458
   Pro Gly Gln Glu Leu Ala Asp Arg Pro Pro Ile Ala Ile Phe Gln Leu
               95                  100                 105
   atc cag cga ggt gga ggc atc ttc ttc tca tct ggg gcg cgc tgg agg      506
   Ile Gln Arg Gly Gly Gly Ile Phe Phe Ser Ser Gly Ala Arg Trp Arg
           110                 115                 120
   gct gcc cgc cag ttc acg gtg cgt gcc ctg cac agc ctg ggc gtg ggc      554
   Ala Ala Arg Gln Phe Thr Val Arg Ala Leu His Ser Leu Gly Val Gly
       125                 130                 135
   cgg gag ccg gtg gct gac aag att ctg cag gag ctg aaa tgc ctc tct      602
   Arg Glu Pro Val Ala Asp Lys Ile Leu Gln Glu Leu Lys Cys Leu Ser
   140                 145                 150                 155
   ggg cag ctg gat ggc tac aga ggc cgg ccc ttc ccg ctg gcc cta ctg      650
   Gly Gln Leu Asp Gly Tyr Arg Gly Arg Pro Phe Pro Leu Ala Leu Leu
                   160                 165                 170
   ggc tgg gct ccc tcc aat atc acc ttc gcg ctc ctc ttc ggc cgc cga      698
   Gly Trp Ala Pro Ser Asn Ile Thr Phe Ala Leu Leu Phe Gly Arg Arg
               175                 180                 185
   ttt gac tac cgg gac ccc gtg ttt gtg tcc ctg ctg ggt ctc atc gat      746
   Phe Asp Tyr Arg Asp Pro Val Phe Val Ser Leu Leu Gly Leu Ile Asp
           190                 195                 200
   gag gtc atg gtc ctc ttg ggg tcc cct ggc ctg cag ctg ttc aac gtc      794
   Glu Val Met Val Leu Leu Gly Ser Pro Gly Leu Gln Leu Phe Asn Val
       205                 210                 215
   tac cca tgg ctc ggg gcc ctg ctc cag ctg cac cgg ccc gtc ctg cgc      842
   Tyr Pro Trp Leu Gly Ala Leu Leu Gln Leu His Arg Pro Val Leu Arg
   220                 225                 230                 235
   aag atc gag gag gtc cgt gcc att ctg agg acc ctc ctg gag gcg cgg      890
   Lys Ile Glu Glu Val Arg Ala Ile Leu Arg Thr Leu Leu Glu Ala Arg
                   240                 245                 250
   agg ccc cac gtg tgc ccg ggg gac ccc gtg tgc agc tat gtg gac gcc      938
   Arg Pro His Val Cys Pro Gly Asp Pro Val Cys Ser Tyr Val Asp Ala
               255                 260                 265
   ctg atc cag cag gga cag ggg gat gac ccc gag ggc ctg ttt gct gag      986
   Leu Ile Gln Gln Gly Gln Gly Asp Asp Pro Glu Gly Leu Phe Ala Glu
           270                 275                 280
   gcc aac gcg gtg gcc tgc acc ctg gac atg gtc atg gcc ggg acg gag     1034
   Ala Asn Ala Val Ala Cys Thr Leu Asp Met Val Met Ala Gly Thr Glu
       285                 290                 295
   acg acc tcg gcc acg ctg cag tgg gcc gca ctt ctg atg ggc cgg cac     1082
   Thr Thr Ser Ala Thr Leu Gln Trp Ala Ala Leu Leu Met Gly Arg His
   300                 305                 310                 315
   ccg gac gtg cag ggc cgg gtg cag gag gag cta gac cgc gtg ctg ggc     1130
   Pro Asp Val Gln Gly Arg Val Gln Glu Glu Leu Asp Arg Val Leu Gly
                   320                 325                 330
   cct ggg cgg act ccc cgg ctg gag gac cag cag gct ctg ccc tac aca     1178
   Pro Gly Arg Thr Pro Arg Leu Glu Asp Gln Gln Ala Leu Pro Tyr Thr
               335                 340                 345
   agc gcc gtg ctc cac gag gtg cag cgg ttc atc acg ctc ctg ccg cac     1226
   Ser Ala Val Leu His Glu Val Gln Arg Phe Ile Thr Leu Leu Pro His
           350                 355                 360
   gtg ccc cgc tgc acc gcg gcc gac aca cag ctg ggc ggc ttc ctg ctc     1274
   Val Pro Arg Cys Thr Ala Ala Asp Thr Gln Leu Gly Gly Phe Leu Leu
       365                 370                 375
   ccc aag ggc acg ccc gtg att ccc ctg ctg acc tcg gtg ctc ctg gat      1322
   Pro Lys Gly Thr Pro Val Ile Pro Leu Leu Thr Ser Val Leu Leu Asp
   380                 385                 390                 395
   gag aca cag tgg cag acc cca ggc cag ttc aac ccc ggc cat ttc ctg      1370
   Glu Thr Gln Trp Gln Thr Pro Gly Gln Phe Asn Pro Gly His Phe Leu
                   400                 405                 410
   gac gcg aat ggg cac ttt gtg aag cgg gag gcc ttc ctg cct ttc tct      1418
   Asp Ala Asn Gly His Phe Val Lys Arg Glu Ala Phe Leu Pro Phe Ser
               415                 420                 425
   gca ggc cgc cgc gtc tgt gtt ggg gag cgc ctg gcc agg acc gag ctc      1466
   Ala Gly Arg Arg Val Cys Val Gly Glu Arg Leu Ala Arg Thr Glu Leu
           430                 435                 440
   ttc ctg ctg ttt gcc ggc ctc ctg cag agg tac cgc ctg ctg ccc ccg      1514
   Phe Leu Leu Phe Ala Gly Leu Leu Gln Arg Tyr Arg Leu Leu Pro Pro
       445                 450                 455
   cct ggc gtc agt ccg gcc tcc ctg gac acc acg ccc gcc cgg gct ttt      1562
   Pro Gly Val Ser Pro Ala Ser Leu Asp Thr Thr Pro Ala Arg Ala Phe
   460                 465                 470                 475
   acc atg agg ccg agg gcc cag gcc ctg tgt gcg gtg ccc agg ccc t        1608
   Thr Met Arg Pro Arg Ala Gln Ala Leu Cys Ala Val Pro Arg Pro
                   480                 485                 490
   aggagctccc ccagccccca ggtcctcctg accactcccc tcccagccct gggtcctccc    1668
   accctctctc ctcccacccc acagctcgga ctgctctggg agggccctga ggactcccac    1728
   cctcaccccc acccccacag ggtcagcaac tgcttccggt tacacccagg actacccctg    1788
   cccgaccctg tgggaccccc acccctctga tgctgtctgc agctcagtcc ctgccagccc    1848
   ccaggagcgc ctccagggcc ccgcccactc tcccacccct gaagctgcac tcccacccac    1908
   ctagctcccc ccagggcccc ccagcaccta cagctggggc tgcagggaga caacgggtgg    1968
   ctgcatccag ccagagacag gcgcaggtgg gtgtcctcag cgtgcgagcc ctgcaccccc    2028
   caggtcctgg gactcctgca gaccccactc cattcccgct cctggaacac ttcctgcagc    2088
   tgtgcctgga ggcagtcggc ctgcagtgcc agactctgag ccaagccact ggggccatgc    2148
   gtatgactgg tgcagggagg caaggcccac attctccttc agagacaggc actggcgcca    2208
   gaggcttcct tggggcgggg ggagggcacc tcagcccctg aagacaagca gcactgcagt    2268
   ggcaaaaatg gaaacactga cccggtgcgg tggctcatgc ctgtaatccc agcactttgg    2328
   gaggccgagg caggcgaatc acgaggtcag gagttcgaga ccagcctgcc caacatggtg    2388
   aaaccctgtc tctactaaaa atacaaaaaa attaaccgag catg                     2432
<210> 10
   <211> 137
   <212> DNA
   <213> Homo sapiens
<220>
   <221> promoter
   <222> (1)..(137)
   <223> -137 to -1 5'UTR of CYP2W1 gene
<400> 10
   gccctgcaaa ctgctcgttc cacattctcg gggtggtggg gtgggtgggg tgcgggcacg 60
   ccctcccgcg gaggcctata agggtgcggg ggggacgggg cccaggaggg gagtggagcc 120
   tcaccagcca cgtcctc 137